# EUROPÄISCHE PATENTSCHRIFT

(11) **EP 1 448 186 B1**
(45) Veröffentlichungstag und Bekanntmachung des Hinweises auf die Patenterteilung: **17.12.2008**
(21) Anmeldenummer: 02803380.1
(22) Anmeldetag: 18.11.2002
(51) Int. Cl.: A61K 31/23, A61K 31/25, A61K 31/353, A61P 17/12, A61P 31/20

(54) **ARZNEIMITTEL ZUR BEHANDLUNG VON VIRALEN HAUT- UND TUMORERKRANKUNGEN**
MEDICAMENT FOR THE TREATMENT OF VIRAL SKIN AND TUMOUR DISEASES
MEDICAMENT POUR TRAITER DES MALADIES DE PEAU VIRALES ET DES MALADIES TUMORALES

(30) Priorität: 19.11.2001 DE 10156794; 19.11.2001 US 331705 P
(43) Veröffentlichungstag der Anmeldung: 25.08.2004
(73) Patentinhaber: MediGene AG, 82152 Planegg (DE)
(72) Erfinder: CHANG, Yunik, Sonoma, CA 95476 (US); LATHROP, Robert, Fort Collins, CA 80526 (US); BÖHM, Erwin, 68526 Ladenburg (DE); GANDER-MEISTERERNST, Irene, 48149 Münster (DE); GREGER, Regina, 82393 Iffeldorf (DE); HOLLDACK, Johanna, 8400 Ebeltoft (DK); MOEBIUS, Ulrich, 82131 Gauting-Unterbrunn (DE)
(74) Vertreter: Bösl, Raphael Konrad
(86) Internationale Anmeldenummer: PCT/EP2002/012919
(87) Internationale Veröffentlichungsnummer: WO 2003/043628

(56) Entgegenhaltungen:
- EP-A- 0 087 161
- EP-A- 0 842 660
- EP-A- 1 005 862
- WO-A-00/29027
- WO-A-00/33832
- WO-A-98/20872
- WO-A-99/66897
- US-A- 4 002 775
- US-A- 5 633 284
- SANDS J ET AL: "Extreme sensitivity of enveloped viruses, including herpes simplex, to long-chain unsaturated monoglycerides and alcohols." ANTIMICROBIAL AGENTS AND CHEMOTHERAPY. UNITED STATES JAN 1979, Bd. 15, Nr. 1, Januar 1979 (1979-01), Seiten 67-73, XP009007372 ISSN: 0066-4804

## Beschreibung

Die vorliegende Erfindung betrifft ein Arzneimittel, enthaltend eine Verbindung der allgemeinen Formel 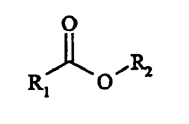 wobei, R₁ ein C₁₃-Alkylrest bedeutet, und
R₂ unabhängig voneinander ein unverzweigter oder verzweigter C₁-C₈ Alkyl Alkylen- oder Alkinylrest, vorzugsweise ein C₁-C₆- Alkyl-, Alkylen- oder Alkinylrest, insbesondere ein C₂-C₄ Alkyl-, Alkylen- oder Alkinylrest, vor allem ein C₃-Alkylrest, ein -[CH₂-(CH₂)ₘ-O]ₙ-H Rest mit n = 1 bis 10, vorzugsweise n = 1 bis 5, m = 1 bis 5, vorzugsweise m = 1 bis 3,
einen -CH₂-[CH-(OH)]ₚ-[CH₂-(R₃)]-Rest, wobei R₃ unabhängig voneinander ein Wasserstoff oder ein Hydroxylrest, p = 1 bis 7, vorzugsweise p = 1 bis 4, ein Pentose-Rest oder ein Hexose-Rest bedeutet, als therapeutisch aktiven Wirkstoff zusammen mit mindestens einem catechin der allgemeinen Formel (IV), als Kombinationspräparat zur Behandlung von viralen Hauterkrankungen und/oder Tumorerkrankungen, hervorgerufen durch humane Papilloma Viren (HPV) sowie eine topisch wirkende Arzneimittelformulierung und deren Verwendung.

Papilloma Viren (HPV) sind DNA-Viren, die die Epithelialzellen von Säugetieren infizieren und dadurch ein unkontrolliertes Zellwachstum hervorrufen. Es gibt die unterschiedlichsten Arten von Papilloma Viren, die Menschen und verschiedene Tierarten infizieren. Alle Arten infizieren hierbei die basalen Epithelialzellen und verbleiben episomal oder integrieren ihre DNA ins Wirtsgenom.

Es ist seit langem bekannt, dass Papilloma Viren Genitalwarzen (Condyloma acuminata), gewöhnliche und plantare Warzen, bowenoide Papulose bei Männern und Frauen, und cervicale intra-epitheliale Neoplasien bei Frauen hervorrufen.

Je nach Methode liegt die Detektionsrate der HPV bei fast 100 %. In Warzen und Genitalwarzen (Condyloma acuminata)werden vor allem HPV 6 und HPV 11 Viren gefunden. Da HPV 16 und HPV 18 vor allem in malignen, schuppigen Zellkarzinomen wie bei Penis- und Gebärmutterhalskrebs beobachtet wird, ist allgemein akzeptiert, dass HPV 16 und HPV 18 in Verbindung mit bösartigen HPV-Erkrankungen steht.

Durch humane Papilloma Viren verursachte Genitalwarzen werden gegenwärtig überwiegend mit physikalischen Methoden behandelt. Hierzu gehören die operative Entfernung, Elektrokauterisierung, Kryochirurgie oder Lasertherapie, um nur einige zu nennen. Eine weitere medizinische Behandlung ist die Anwendung von Podophyllin, 5-Fluoruracil, Bleomycin, Interferon, Imiquimod, usw.

Die operative Behandlung hat den Nachteil, dass sie für den Patienten sehr unangenehm ist und zu weiteren Infektionen führen kann. Bei einer topischen Anwendung bestand bisher das Risiko von Nebenwirkungen, da die verwendeten Wirkstoffe cytotoxische Eigenschaften besitzen oder die zellulare Immunabwehr verstärken und somit lokale Entzündungen hervorrufen können. Dies zeigt, dass die bisher vorhandenen Therapiemöglichkeiten noch nicht zufriedenstellend sind.

Hinzu kommt, dass die Rückfallquote bei Warzen und Genitalwarzen sehr hoch ist und eine komplette Heilung nur durch konstante und konsequente Behandlung möglich ist. Aus diesem Grund besteht der Wunsch nach einer sicheren und bequemen Behandlung.

Besonders bei der Behandlung von Gentialwarzen, aber auch bei allen übrigen Krankheiten, die durch das Papilloma Virus hervorgerufen werden, ist eine Behandlung erwünscht, die für den Patienten leicht anwendbar ist. Geeignet wäre beispielweise eine Behandlung die der Patient selbst bei den betroffenen Stellen anwenden kann und die in relativ kurzer Zeit gute Resultate zeigt und nur geringe bzw. keine Nebenwirkungen zeigt.

Herpes Simplex Viren (HSV) sind DNA-Viren aus der Alphasubfamilie Herpetoviridae. Sie sind in zwei Gruppen eingeteilt, den HSV 1, dem sogenannten oralen Stamm und den HSV 2, dem sogenannten genitalen Stamm. Herpes Simplex Viren penetrieren als Nukleokapsid in die Nervenendigungen und gelangen mit dem axonalen Strom in die zugehörigen Ganglien. Sie werden durch Schmier- und Tröpfcheninfektion aus Herpesläsionen oder von gesunden Dauerausscheidern übertragen. Nach einer Primärinfektion kann es zu einer erneuten, symptomatischen oder asymptomatischen Reaktivierung der Viren durch eine Irritation, z. B. durch Fieber, Traumata oder Strahlung, von latent infizierten Neuronen kommen. Diese Reaktivierung hängt insbesondere von der Abwehrlage des gesamtem Organismus ab. Herpes Simples Viren können Zellen in Tieren und Zellkulturen neoplastisch transformieren. Es wird derzeit ein Zusammenspiel von Herpes Simplex Viren vom Typ 2 und der Genese von Zervixkarzinomen mit den humanen Papilloma Viren vom Typ 16 und 18 diskutiert.

Eine Behandlung von Herpesinfektionen durch Behandlung mit einer Mischung aus Isopropylmyristat und kleinen Mengen von 4-{Niebrigalkyl}-2,6-(bis-*tert-*Butyl)-Phenol mit 2,6-(bis-*tert*-Butyl)-4-Hydroxy-Toluol bzw. 4-(Niedrigalkyl)-2,6-(bis-*tert*-Butyl)-Phenol ist bereits aus der EP 0 087 161 bekannt.

In der EP 0 842 660 ist eine pharmazeutische Zusammensetzung zur Behandlung von Genitalwarzen, die durch humane Papilloma Viren verursacht werden, beschrieben. Die beschriebene Zusammensetzung enthält Catechine aus Tee-Extrakten (Camellia sinensis), überwiegend (-)-Epigallocatechin-gallat in Form einer Salbe oder eines Zäpfchens.

Aufgabe der vorliegenden Erfindung ist daher, weitere wirksame antivirale Substanzen und Formulierungen zu finden, die zur Behandlung von viralen Hauterkrankungen und/oder Tumorerkrankungen, hervorgerufen durch Papilloma Viren geeignet sind.

Überraschenderweise wurde nun gefunden, dass ein Arzneimittels, enthaltend eine anspruchsgemäße Verbindung der allgemeinen Formel (I) als pharmazeutisch aktiven Wirkstoff, dazu geeignet ist, virale Hauterkrankungen und/oder Tumorerkrankungen zu behandeln.

Die vorliegende Erfindung betrifft somit die Verwendung einer Verbindung der allgemeine Formel (I) als pharmazeutisch aktiven Wirkstoff,

A-B (I)

wobei A ein Rest der allgemeinen Formel (II) 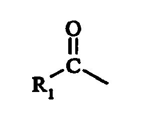 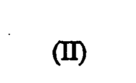 und B ein Rest der allgemeinen Formel (III)

-O-R₂ (III)

bedeutet, und
R₁ ein C₁₃-Alkylrest bedeutet, und
R₂ unabhängig voneinander ein unverzweigter oder verzweigter C₁-C₈ Alkyl Alkylen- oder Alkinylrest, vorzugsweise ein C₁-C₆- Alkyl-, Alkylen- oder Alkinylrest, insbesondere ein C₂-C₄ Alkyl-, Alkylen- oder Alkinylrest, vor allem ein C₃-Alkylrest, ein -[CH₂-(CH₂)ₘ-O]ₙ-H Rest mit n = 1 bis 10, vorzugsweise n = 1 bis 5, m = 1 bis 5, vorzugsweise m = 1 bis 3,
einen -CH₂-[CH-(OH)]ₚ-[CH₂-(R₃)]-Rest, wobei R₃ unabhängig voneinander ein Wasserstoff oder ein Hydroxylrest, p = 1 bis 7, vorzugsweise p = 1 bis 4, ein Pentose-Rest oder ein Hexose-Rest bedeutet,
und mindestens ein Catechin der allgemeinen Formel (IV) zur Herstellung eines Arzneimittels mit mindestens 5% - 75% (w/w), vorzugsweise mindestens 10% -60%(w/w), insbesondere mindestens 25%-55% (w/w), und vor allem mindestens 35%-50% (w/w) der Verbindung (I) zur Behandlung von viralen Hauterkrankungen und/oder Tumorerkrankungen, die durch Papilloma Viren hervorgerufen werden.

Der Rest R₁ und oder der Rest R₂ können dabei unabhängig voneinander mit einem Halogen, vorzugsweise Fluor und/oder Chlor, einem unverzweigten oder verzweigten C₁-C₆- Alkyl-, Alkylen- oder Alkinylrest, vorzugsweise einem C₁-C₃-Alkyl-, Alkylen- oder Alkinylrest, insbesondere einem Methylrest substituiert sind.

Der Rest A der Verbindung (I) ist abgeleitet von Myristinsäure.

Der Rest B der Verbindung (I) kann beispielsweise abgeleitet sein von einem unverzweigten oder verzweigten C₁-C₈ Alkylalkohol, insbesondere Ethanol, Propanol, Isopropanol, n-Butanol, *tert*-Butanol besonders bevorzugt Isopropanol, Ethylenglykol, Polyethylenglykol, Propylenglykol, Polypropylenglykol, Glycerin, Polyglycerin, einem Pentose-Zucker wie beispielsweise Arabit, Adonit und Xylit, oder einem Hexose-Zucker wie beipsielsweise Sorbit, Mannit oder Dulcit, bevorzugt von Ethanol, Propanol, Isopropanol, Butanol, Ethylenglykol, Polyethylenglykol, Propylenglykol, Polyproylenglykol, Glycerin, Polyglycerin, Arabit, Adonit, Xylit, Sorbit, Mannit, und/oder Dulcit

Die Verbindung (I) ist bevorzugt Isopropylmyristat.

In einer weiteren bevorzugten Ausführungsform ist die Verbindung (I) eine hydrophobe Verbindung. Unter einer hydrophoben Verbindung wird gemäß der vorliegenden Erfindung, eine Verbindung verstanden, deren Löslichkeit in Wasser maximal ca. 0,2 mg/ml, insbesondere maximal ca. 0,1 mg/ml beträgt.

Das Arzneimittel enthält beispielsweise mindestens ca. 5 % - 75 % (w/w), vorzugsweise mindestens ca. 10 % - 60 % (w/w), insbesondere mindestens ca. 25 % - 55 % (w/w), und vor allem mindestens ca. 35 % - 50 % (w/w) der Verbindung der allgemeinen Formel (I).

Das Arzneimittel enthält ein Catechin der allgemeinen Formel (IV) 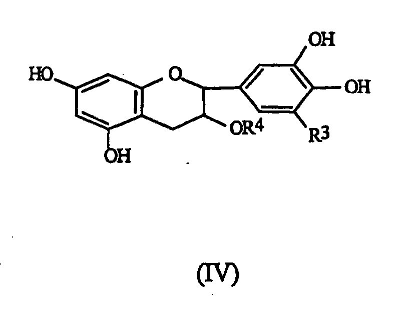 worin,
R₃ ein -H oder -OH und
R₄ ein -H oder eine Gruppe der Formel (V) 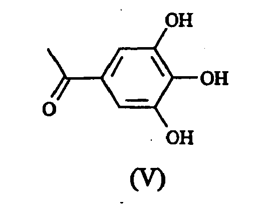 bedeutet.

Die hierbei zugesetzten Catechine können entweder synthetisch oder aus natürlichen Quellen gewonnen werden. Als natürliche Quellen sind vor allem Teesträucher zu nennen. Je nach Art und Sorte können hier die natürlichen Bestandteile in unterschiedlichen Konzentrationen vorhanden sein. Hierbei werden die verwendeten Catechine bevorzugt aus Camellia sinensis, Camellia asamica, Camellia bohea, Camellia chinensis oder Camellia oleosa gewonnen. Zur Gewinnung der Catechine können sämtliche Bestandteile von Teesträuchern, insbesondere die Blätter, verwendet werden. Bevorzugt werden die verwendeten Catechine aus einem Teeextrakt gewonnen.

Die verwendeten Catechine der vorliegenden Erfindung sind bevorzugt Epicatechin, Epicatechin Gallat, Epigallocatechin, Epigallocatechin Gallat, Gallocatechin und Gallocatechin Gallat, insbesondere (-)-Epicatechin, (-)-Epicatechin Gallat, (-)-Epigallocatechin, (-)-Epigallocatechin Gallat, (+)-Gallocatechin und (-)-Gallocatechin Gallat.

Die Catechine können sowohl einzeln, als auch in Form von Gemischen mit unterschiedlichen Zusammensetzungen verwendet werden. Ein Catechingemisch enthält ca. 2-20 % (w/w), vorzugsweise ca. 4-15 % (w/w), insbesondere ca. 10-11 % (w/w) (-)-Epicatechin, ca. 2-20 % (w/w), vorzugsweise ca. 5-15 % (w/w), insbesondere ca. 5-7% (w/w) (-)-Epicatechin Gallat, ca. 1-25 % (w/w), vorzugsweise ca. 3-15% (w/w), insbesondere ca. 5-7 % (w/w) (-)-Epigallocatechin, ca. 40-75 % (w/w), vorzugsweise ca. 57-67 % (w/w), insbesondere ca. 61-66 % (w/w) (-)-Epigallocatechin Gallat, ca. 0,05-5 % (w/w), vorzugsweise ca. 0,1-1 % (w/w), insbesondere ca. 0,1-0,6 % (w/w) (+)-Gallocatechin und/oder ca. 0,5-20 % (w/w), vorzugsweise ca. 1-10 % (w/w), insbesondere ca. 1-5 % (w/w) (-)-Gallocatechin Gallat.

In einer bevorzugten Ausführungsform besteht das Catechingemisch aus ca. 5,9 % (w/w) (-)-Epicatechin, ca. 12,6 % (w/w) (-)-Epicatechin Gallat, ca. 17,6 % (w/w) (-)-Epigallocatechin, ca. 53,9 % (w/w) (-)-Epigallocatechin Gallat und/oder ca. 1,4 % (w/w) (-)-Gallocatechin. Eine derartige Zusammensetzung ist unter dem Namen Polyphenon®100 bekannt.

In einer besonders bevorzugten Ausführungsform besteht das Catechingemisch aus ca. 10,8 % (w/w) (-)-Epicatechin, ca. 6,5 % (w/w) (-)-Epicatechin Gallat, ca. 9,2 % (w/w) (-)-Epigallocatechin, ca. 54,8 % (w/w) (-)-Epigallocatechin Gallat und/oder ca. 4,0 % (w/w) (-)-Gallocatechin Gallat Eine derartige Zusammensetzung ist unter dem Namen Polyphenon® E bekannt.

Das erfindungsgemäße Arzneimittel enthält beispielsweise ca. 1-30 % (w/w), vorzugsweise ca. 2-20 % (w/w) und insbesondere ca. 15-18 % (w/w) eines Catechins sowie mindestens ca. 5-90 % (w/w), vorzugsweise mindestens ca. 10-70 % (w/w), insbesondere mindestens ca. 25-60 % (w/w) und vor allem mindestens ca. 35-50 % (w/w) der Verbindung (I).

Die Herstellung von Arzneimitteln mit einem Gehalt an einer oder mehreren erfindungsgemäßen Verbindungen bzw. deren Einsatz bei der erfindungsgemäßen Verwendung erfolgt in üblicher Weise anhand geläufiger pharmazeutischtechnologischer Verfahren. Dazu werden die Wirkstoffe zusammen mit geeigneten, pharmazeutisch annehmbaren Hilfs- und Trägerstoffen zu den für die verschiedenen Indikationen und Applikationsorte geeigneten Arzneiformen verarbeitet. Dabei können die Arzneimittel in der Weise hergestellt werden, dass die jeweils erwünschte Freisetzungsrate, z.B. eine rasche Anflutung und/oder ein Retard- bzw. Depoteffekt, erzielt wird.

Zur herkömmlichen Applikation auf der Haut oder Schleimhaut lassen sich übliche Emulsionen, Gele, Salben, Cremes der mischphasigen bzw. amphiphilen Emulsionssysteme (Öl/Wasser-Wasser/Öl-Mischphase) sowie Liposomen und Transfersomen oder Pflaster, bevorzugt Salben und Cremes, besonders bevorzugt eine Salbe anführen. Vorzugsweise wird der Wirkstoff lokal in dem Bereich appliziert, in dem eine Haut- bzw. Schleimhautveränderung und/oder -erkrankung vorliegt.

Als topisch, lokal oder regional verabreichbare Arzneimittel kommen neben den bekannten Anwendungen auf der Haut und/oder Schleimhaut als spezielle Zubereitungen die folgenden in Betracht: genital, vaginal oder rektal, insbesondere genital und vaginal applizierbare Emulsionen, Cremes, Salben, Schaumtabletten oder Suppositorien. Auf der Grundlage von Gelatine oder anderen Trägerstoffen lassen sich auch Rektalkapseln bereitstellen. Als Suppositoriengrundlagen kommen Hartfette, wie z.B. Witepsol®, Massa Estarium®, Novata®, Kokosfett, Glycerin/Gelatine-Massen, Glycerin/Seifen-Gele und Polyethylenglykole in Betracht.

Als Hilfs- bzw. Trägerstoffe eignen sich beispielsweise Natriumalginat als Gelbildner zur Herstellung einer geeigneten Grundlage oder Cellulosederivate, wie z. B. Guar- oder Xanthangummi, anorganische Gelbildner, wie z.B. Aluminiumhydroxide oder Bentonite (sog. thixotrope Gelbildner), Polyacrylsäurederivate, wie z.B. Carbopol®, Polyvinylpyrrolidon, mikrokristalline Cellulose oder Carboxymethylcellulose. Weiterhin kommen amphiphile nieder- und höhermolekulare Verbindungen sowie Phospholipide in Betracht. Die Gele können entweder als Hydrogele auf Wasserbasis oder als hydrophobe Organogele, beispielsweise auf Basis von Gemischen nieder- und hochmolekularer Paraffinkohlenwasserstoffe und Vaseline vorliegen. Die hydrophilen Organogele können beispielsweise auf Basis hochmolekularer Polyethylenglykole zubereitet werden. Diese gelartigen Formen sind abwaschbar. Unter den Organogelen sind die hydrophoben Organogele jedoch bevorzugt. Besonders bevorzugt sind hydrophobe Hilfsstoffe- und Zusatzstoffe wie Petrolatum, Wachs, Oleylalkohol, Propylenglykolmonostearat und Propylenglykolmonopalmitostearat. Dem Fachmann bekannte hautberuhigende und/oder endzündungshemmende Zusatzstoffe wie beispielsweise synthetisch hergestellte Wirkstoffe und /oder Extrakte und /oder Wirkstoffe aus Heilpflanzen, insbesondere Bisobolol und Panthenol können selbstverständlich ebenso zugesetzt werden. Des weiteren können Farbstoffe beispielsweise gelbes und/oder rotes Eisenoxid und /oder Titandioxid zur farblichen Anpassung hinzugegeben werden.

Als Emulgatoren lassen sich anionische, kationische oder neutrale Tenside, beispielsweise Alkaliseifen, Metallseifen, Aminseifen, sulfurierte und sulfonierte Verbindungen, Invertseifen, hohe Fettalkohole, Partialfettsäureester des Sorbitants und Polyoxyethylensorbitans, z.B. Lanette-Typen, Wollwachs, Lanolin oder andere synthetische Produkte zur Herstellung der Öl/Wasser- und /oder Wasser/Öl-Emulsionen einsetzen.

Als Lipide in Form fett- und/oder öl- und/oder wachsartiger Komponenten zur Herstellung der Salben, Cremes oder Emulsionen können Vaseline, natürliche oder synthetische Wachse, Fettsäuren, Fettalkohole, Fettsäureester, z.B. als Mono-, Di- oder Triglyceride, Paraffinöl oder vegetabilische Öle, gehärtetes Rizinusöl oder Kokosöl, Schweinefett, synthetische Fette, z.B. auf Caprryl-, Caprin-, Laurin- und Stearinsäurebasis wie z.B. Softisan® oder Triglyceridgemische wie Miglyol® eingesetzt werden.

Zur Einstellung des pH-Wertes können beispielsweise osmotisch wirksame Säuren und Laugen, z.B. Salzsäure, Citronensäure, Natronlauge, Kalilauge, Natriumhydrogencarbonat, ferner Puffersysteme, wie z.B. Citrat, Phosphat, Tris-Puffer oder Triethanolamin verwendet werden.

Zur Erhöhung der Stabilität können noch Konservierungsmittel, wie beispielsweise Methyl- oder Propylbenzoat (Parabene) oder Sorbinsäure hinzugesetzt werden.

Als weitere topisch applizierbare Formen lassen sich Pasten, Puder oder Lösungen erwähnen. Die Pasten enthalten als konsistenzgebende Grundlagen oft hydrophobe und hydrophile Hilfsstoffe, bevorzugt jedoch hydrophobe Hilfsstoffe mit sehr hohem Feststoffanteil. Die Puder oder topisch applizierbare Pulver können zur Erhöhung der Dispersität sowie des Fließ- und Gleitvermögens sowie zur Verhinderung von Agglomeraten, z.B. Stärkearten, wie Weizen- oder Reisstärke, flammendisperses Siliziumdioxid oder Kieselerden, die auch als Verdünnungsmittel dienen, enthalten.

Die jeweils geeigneten Arzneiformen lassen sich in Einklang mit dem Fachmann bekannten Rezepturvorschriften und Verfahrensweisen auf der Basis pharmazeutisch-physikalischer Grundlagen herstellen.

Das erfindungsgemäße Arzneimittel enthält bevorzugt ca. 35 % (w/w) Isopropylmyristat, ca. 15 % (w/w) mindestens eines Catechins, ca. 24,5 % (w/w) Petrolatum, ca. 20 % (w/w) Wachs, ca. 5 % (w/w) Propylenglykolmonostearat oder Propylenglykolmonopalmitostearat und ca. 0,5 % (w/w) Oleylalkohol.

Das Arzneimittel der vorliegenden Erfindung und /oder eines pharmazeutischen Metaboliten findet seine Anwendung in der Behandlung von viralen Hauterkrankungen und/oder Tumorerkrankungen.

Unter dem Begriff pharmazeutischer Metabolit sind eine oder mehrere Verbindungen zu verstehen, die durch den biologischen Stoffwechsel während der Anwendung entstehen. Es kann sich hierbei um Zwischenprodukte während des intermediären Stoffwechsels oder um Endprodukte des Stoffwechsels handeln. Bevorzugt handelt es sich hierbei um Stoffwechselprodukte, die durch Auftragen auf die Haut und/oder Schleimhaut entstehen, insbesondere Hydrolyseprodukte der Verbindung mit der allgemeinen Formel (I). Denkbare Hydrolyseprodukte können beispielsweise von Rest A und/oder Rest B abgeleitet sein.

Unter viralen Hauterkrankungen werden Hauterkrankungen verstanden, die von Viren ausgelöst bzw. verursacht und/oder mit Virusinfektionen assoziiert sind. Hierzu zählen beispielsweise Hauterkrankungen wie Warzen, Genitalwarzen, durch Papilloma Viren verursachte benigne Tumoren der Haut und/oder Schleimhaut, zum Beispiel Verrucae plantares, Verrucae vulgares, Verrucae planae juveniles, Epidermodysplasia verrucifomis, Condylomata acuminata, Condylomata plana, bowenoide Papulose, Papillome an Larynx und Mundschleimhaut oder fokale epitheliale Hyperplasie,

Diese viralen Hauterkrankungen und/oder Tumorerkrankungen werden von einem Papilloma Virus bzw. Viren, insbesondere humane Papilloma Viren, wie zum Beispiel HPV 1, 2, 3, 4, 5, 6, 8, 9, 11, 12, 13, 14, 15, 16, 17, 18, 19-29, 31, 32, 34, 36-38, 46-50, 56, 58 hervorgerufen.

Die Figuren und die folgenden Beispiele sollen die Erfindung näher erläutern, ohne sie zu beschränken. Die Fachwelt kann im Rahmen des üblichen Könnens die Erfindung entsprechend modifizieren, ohne vom Schutzumfang abzuweichen.

### Beispiele

### Klinische Studie zum Vergleich von einer Salbe und einer Creme enthaltend Isopropylmyristat

In einer multizentrischen klinischen Studie, die an insgesamt 30 verschiedenen Zentren in Deutschland und Rußland durchgeführt wurde, nahmen 93 Patienten teil (jeweils zur Hälfte Männer und Frauen). Die Studie war randomisiert und doppel-verblindet. In der Studie wurde die klinische Wirksamkeit von zwei verschiedenen Formulierungen von Isopropylmyristat (eine Salbe und eine Creme) bei der Behandlung von äußerlichen Genitalwarzen geprüft.

Die getesteten Formulierungen hatten die folgende Zusammensetzung:

### Creme 1:

| **Substanz** | **Menge (in % w/w)** |
|---|---|
| Cera alba | 6,996 |
| Monomuls | 2,798 |
| Lameform TGI | 5,598 |
| Cetiol V | 6,996 |
| Isopropylmyristat | 13,992 |
| Tocopherol | 0,699 |
| Controx KS | 0,066 |
| Glycerin | 6,996 |
| Disodium EDTA | 0,001 |
| Magnesiumsulfate | 1,399 |
| D-Panthenol | 0,699 |
| purified water | 53,678 |
| Red Iron Oxide* | 0,025 |
| Yellow Iron Oxide* | 0,054 |

| | |
|---|---|
| *Die Farbstoffe wurden zur farblichen Anpassung hinzugegeben. | |

### Salbe 1:

| **Substanz** | **Menge (in % w/w)** |
|---|---|
| White Petrolatum, USP | 34,023 |
| White Wax,NF | 25,000 |
| Isopropyl Myristate, NF | 35,000 |
| Oleyl Alcohol, NF | 0,500 |
| Propylene Glycol Monostearate, NF | 5,000 |
| Red Iron Oxide* | 0,022 |
| Yellow Iron Oxide* | 0,055 |
| Titanium Dioxide, USP* | 0,400 |

| | |
|---|---|
| *Die Farbstoffe wurden zur farblichen Anpassung hinzu gegeben. | |

Die Patienten trugen die topische Studienmedikation dreimal täglich oder bis zur vollständigen Heilung der Genitalwarzen oder für maximal 12 Wochen auf.

Während der Studie wurden die folgenden Daten erhoben:

### Vollständige Heilung (in %)

| | **Creme 1** | **Salbe 1** |
|---|---|---|
| männlich | 39,1 | 42,1 |
| weiblich | 35,0 | 33,3 |

Teilweise Heilung (in %); diese entspricht mindestens 75% Heilung bezogen auf die Gesamtfläche der Genitalwarzen.

| | **Creme 1** | **Salbe 1** |
|---|---|---|
| männlich | 43,5 | 63,2 |
| weiblich | 50,0 | 52,4 |

Die Ergebnisse der Studie zeigen, daß im Vergleich zu Plazebo-Werten aus ähnlichen Studien mit unterschiedlichen Formulierungen, bei denen die spontanen Regression von Genitalwarzen bei ca. 20% bei Frauen und ca. 5% bei Männern liegt (Aldara^{™} (Imiquimod) Cream, 5% Product Monograph, vertrieben durch 3M Pharmaceuticals, Northridge, CA, Beutner KR et al. (1998) J Am Acad Dermatol 38, 230-9, Edwards L et al. (1998) Arch Dermatol 134 (1); 25-30) eine überraschend hohe vollständige Heilung bzw. teilweise Heilung erfolgt.

Dieser therapeutische Effekt wird dem Isopropylmyristat zugeschrieben, für das somit erstmals eine antivirale Wirkung gezeigt werden konnte.

### Klinische Studie zum Vergleich von einer Salbe und einer Creme enthaltend Isopropylmyristat und Polyphenon® E

In einer multizentrischen klinischen Studie, die an insgesamt 30 verschiedenen Zentren in Deutschland und Rußland durchgeführt wurde, nahmen 272 Patienten teil (jeweils zur Hälfte Männer und Frauen). Die Studie war randomisiert und doppel-verblindet. In der Studie wurde die klinische Wirksamkeit von zwei verschiedenen Formulierungen von Isopropylmyristat und Polyphenon® E (eine Salbe und eine Creme) gegen die Formulierungen aus Beispiel 1 enthaltend Isopropylmyristat bei der Behandlung von äußerlichen Genitalwarzen geprüft.

Die getesteten Formulierungen mit Polyphenon® E hatten die folgende Zusammensetzung:

### Creme 2:

| **Substanz** | **Menge (in % w/w)** |
|---|---|
| Polyphenon® E | 10,000 |
| Cera alba | 5,263 |
| Monomuls | 2,105 |
| Lameform TGI | 4,211 |
| Cetiol V | 5,263 |
| Isopropylmyristat | 10,526 |
| Tocopherol | 0,526 |
| Controx KS | 0,050 |
| Glycerin | 5,263 |
| Disodium EDTA | 0,001 |
| Magnesiumsulfate | 1,053 |
| D-Panthenol | 0,526 |
| purified water | 55,213 |

### Salbe 2:

| **Substanz** | **Menge (in % w/w)** |
|---|---|
| Polyphenon® E | 15,000 |
| White Petrolatum, USP | 24,500 |
| White Wax,NF | 20,000 |
| Isopropyl Myristate, NF | 35,000 |
| Oleyl Alcohol, NF | 0,500 |
| Propylene Glycol Monostearate, NF | 5,000 |

Die Patienten trugen die topische Studienmedikation dreimal täglich oder bis zur vollständigen Heilung der Genitalwarzen oder für maximal 12 Wochen auf.

Während der Studie wurden die folgenden Daten erhoben:

### Vollständige Heilung (in %)

| | **Creme 1** | **Creme 2** | **Salbe 1** | **Salbe 2** |
|---|---|---|---|---|
| männlich | 39,1 | 53,9 | 42,1 | 61,0 |
| weiblich | 35,0 | 39,5 | 33,3 | 56,8 |

### Teilweise Heilung (≥75% in %)

| | **Creme 1** | **Creme 2** | **Salbe 1** | **Salbe 2** |
|---|---|---|---|---|
| männlich | 43,5 | 64,2 | 63,2 | 80,5 |
| weiblich | 50,0 | 47,4 | 52,4 | 81,1 |

Die Auswertung der Studie zeigt im Vergleich, daß zwischen Salbe 1 und Creme 1 auf der einen Seite sowie Salbe 2 und Creme 2 auf der anderen Seite die Kombination von Isopropylmyristat und Polyphenon® E zu einer überraschenden Steigerung der Wirksamkeit des Arzneimittels führt.

Vergleicht man nun die Wirksamkeit von Salbe 2 mit der Creme 2, so wird deutlich, daß die Salbe 2 deutlich wirksamer ist als die Creme 2. Dies deutet auf einen synergistischen Effekt zwischen dem Polyphenon® E und dem Isopropylmyristat in der hydrophoben Salbe hin.

Durch diese synergistische Wirkung können die einzelnen Wirkstoffe in der Formulierung in wesentlich geringeren Mengen eingesetzt werden, als die entsprechenden Einzelkomponenten um die selbe Wirkung zu erzielen. Somit hat der Einsatz dieser synergistischen Formulierung nicht nur Vorteile bezüglich der Wirkung, sondern auch bezüglich der Herstellungskosten für diese Formulierung, was sich wiederum positiv auf die Behandlungskosten des Patienten auswirken kann.

## Patentansprüche

1. Verwendung einer Verbindung der allgemeinen Formel (I) als pharmazeutisch aktiven Wirkstoff,
A-B (I)
und mindestens ein Catechin der allgemeinen Formel (IV), 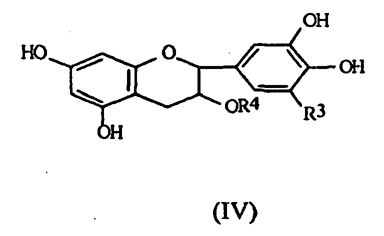 **dadurch gekennzeichnet, dass** A ein Rest der allgemeinen Formel (II) 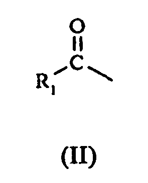 und B ein Rest der allgemeinen Formel (III)
-O-R₂ (III)
bedeutet, und
R₁ ein C₁₃-Alkylrest bedeutet, und
R₂ unabhängig voneinander ein unverzweigter oder verzweigter C₁-C₈ Alkyl Alkylen- oder Alkinylrest, vorzugsweise ein C₁-C₆- Alkyl-, Alkylen- oder Alkinylrest, insbesondere ein C₂-C₄ Alkyl-, Alkylen- oder Alkinylrest, vor allem ein C₃-Alkylrest, ein -[CH₂-(CH₂)ₘ-O]ₙ-H Rest mit n = 1 bis 10, vorzugsweise n = 1 bis 5, m = 1 bis 5, vorzugsweise m = 1 bis 3,
einen -CH₂-[CH-(OH)]ₚ-[CH₂-(R₃)]-Rest, wobei R₃ unabhängig voneinander ein Wasserstoff oder ein Hydroxylrest, p = 1 bis 7, vorzugsweise p = 1 bis 4, ein Pentose-Rest oder ein Hexose-Rest bedeutet, und
dass in Formel (IV)
R₃ ein -H oder -OH und
R₄ ein -H oder eine Gruppe der Formel (V) 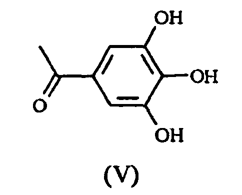 bedeutet,
zur Herstellung eines Arzneimittels mit mindestens 5 % - 75 % (w/w), vorzugsweise mindestens 10 % - 60 % (w/w), insbesondere mindestens 25 % - 55 % (w/w), und vor allem mindestens 35 % - 50 % (w/w) der Verbindung der allgemeinen Formel (I) zur Behandlung von viralen Hauterkrankungen und/oder Tumorerkrankungen, die durch Papilloma Viren hervorgerufen werden.

2. Verwendung nach Anspruch 1, **dadurch gekennzeichnet, dass** unabhängig voneinander der Rest R₁ und/oder der Rest R₂ mit einem Halogen, vorzugsweise Fluor und/oder Chlor, einem unverzweigten oder verzweigten C₁-C₆-Alkyl-, Alkylen- oder Alkinylrest, vorzugsweise einem C₁-C₃- Alkyl-, Alkylen- oder Alkinylrest, insbesondere einem Methylrest substituiert ist.

3. Verwendung nach Anspruch 1 oder 2, **dadurch gekennzeichnet, dass** der Rest A abgeleitet ist von Myristinsäure.

4. Verwendung nach Anspruch 1 oder 2, **dadurch gekennzeichnet, dass** der Rest B abgeleitet ist von Ethanol, Propanol, Isopropanol, Butanol, Ethylenglykol, Polyethylenglykol, Propylenglykol, Polyproylenglykol, Glycerin, Polyglycerin, Arabit, Adonit, Xylit, Sorbit, Mannit, und/oder Dulcit.

5. Verwendung nach mindestens einem der Ansprüche 1-4, **dadurch gekennzeichnet, dass** die Verbindung (I) Isopropylmyristat ist.

6. Verwendung nach mindestens einem der Ansprüche 1-5, **dadurch gekennzeichnet, dass** das Catechin ausgewählt ist aus Epicatechin, Epicatechin Gallat, Epigallocatechin, Epigallocatechin Gallat, Gallocatechin und Gallocatechin Gallat, insbesondere (-)-Epicatechin, (-)-Epicatechin Gallat, (-)-Epigallocatechin, (-)-Epigallocatechin Gallat, (+)-Gallocatechin und (-)-Gallocatechin Gallat.

7. Verwendung nach mindesten einem der Ansprüche 1-6, **dadurch gekennzeichnet, dass** Catechine in Form eines Gemisches vorliegen, bevorzugt enthaltend 5,9 % (w/w) (-)-Epicatechin, 12,6 % (w/w) (-)-Epicatechin Gallat, 17,6 % (w/w) (-)-Epigallocatechin, 53,9 % (w/w) (-)-Epigallocatechin Gallat und 1,4 % (w/w) (-)-Gallocatechin.

8. Verwendung nach mindestens einem der Ansprüche 1-6, **dadurch gekennzeichnet, dass** Catechine in Form eines Gemisches vorliegen, enthaltend 10,8 % (w/w) (-)-Epicatechin, 6,5 % (w/w) (-)-Epicatechin Gallat, 9,2 % (w/w) (-)-Epigallocatechin, 54,8 % (w/w) (-)-Epigallocatechin Gallat und 4,0 % (w/w) (-)-Gallocatechin Gallat.

9. Verwendung nach mindestens einem der Ansprüche 1-8, **dadurch gekennzeichnet, dass** die genannten Catechine aus einem Teeextrakt gewonnen werden.

10. Verwendung nach mindestens einem der Ansprüche 1-9, **dadurch gekennzeichnet, dass** die Formulierung 1-30 % (w/w), vorzugsweise 2-20 % (w/w) und insbesondere 15-18 % (w/w) eines Catechins sowie mindestens 5-90 % (w/w), vorzugsweise mindestens 10-70 % (w/w), insbesondere mindestens 25-60 % (w/w) und vor allem mindestens 35-50 % (w/w) der Verbindung (I) enthält.

11. Verwendung nach mindestens einem der Ansprüche 1-10, **dadurch gekennzeichnet, dass** der pharmazeutische aktive Wirkstoff hydrophob ist.

12. Verwendung nach mindestens einem der Ansprüche 1-11, enthaltend weitere Zusatz- und/oder Hilfsstoffe.

13. Verwendung nach Anspruch 12, **dadurch gekennzeichnet, dass** die Zusatz-und/oder Hilfsstoffe hydrophob sind, vorzugsweise ausgewählt aus Petrolatum, Wachs, Oleylalkohol, Propylenglykolmonostearat oder Propylenglykolmonopalmitostearat.

14. Verwendung nach einem der Ansprüche 1-13, **dadurch gekennzeichnet, dass** das Arzneimittel 35 % (w/w) Isopropylmyristat, 15 % (w/w) mindestens eines Catechins, 24,5 % (w/w) Petrolatum, 20 % (w/w) Wachs, 5 % (w/w) Propyleneglykolmonostearat oder Propylenglykolmonopalmitostearat und 0,5 % (w/w) Oleylalkohol enthält.

15. Verwendung eines Arzneimittels nach einem der Ansprüche 1-14, **dadurch gekennzeichnet, dass** die viralen Hauterkrankungen und/oder Tumorerkrankungen hervorgerufen werden durch humane Papilloma Viren, wie zum Beispiel HPV 1, 2, 3, 4, 5, 6, 8, 9, 11, 12, 13, 14, 15, 16, 17, 18, 19-29, 31, 32, 34, 36-38, 46-50, 56, 58.

16. Verwendung eines Arzneimittels nach Anspruch 15, **dadurch gekennzeichnet, dass** die Hauterkrankungen Warzen, Genitalwarzen, durch Papilloma Viren verursachte benigne Tumoren der Haut und/oder Schleimhaut, zum Beispiel Verrucae plantares, Verrucae vulgares, Verrucae planae juveniles, Epidermodysplasia verrucifomis, Condylomata acuminata, Condylomata plana, bowenoide Papulose, Papillome an Larynx und Mundschleimhaut oder fokale epitheliale Hyperplasie sind.

17. Verwendung eines Arzneimittels nach mindestens einem der Ansprüche 1-16, **dadurch gekennzeichnet, dass** das Arzneimittel topisch, insbesondere genital oder vaginal appliziert wird.

## Claims

1. The use of a compound of the formula (I) as pharmaceutically active compound
A - B (I)
and at least one catechol of the formula (IV) 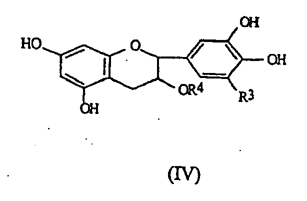 **characterized in that** A is a radical of the formula (II) 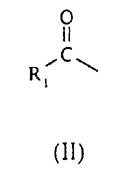 or B is a radical of the formula (III)
-O-R₂ (III),
and
R₁ is a C₁₃-alkyl radical, and
R₂ is, independent of each other, an unbranched or branched C₁-C₈alkyl alkylene or alkynyl radical, preferably a C₁-C₆alkyl, alkylene or alkynyl radical, in particular a C₂-C₄alkyl, alkylene or alkynyl radical, especially a C₃-alkyl radical, a - [CH₂-(CH₂)ₘ-O]ₙ-H radical where n = 1 to 10, preferably n = 1 to 5, m = 1 to 5, preferably m = 1 to 3,
a -CH₂-[CH-(OH)]ₚ-(CH₂-(R₃)] radical, where R₃ is, independent of each other, a hydrogen or a hydroxyl radical, p = 1 to 7, preferably p = 1 to 4, a pentose radical or a hexose radical, and **in that** in formula (IV)
R3 is -H or -OH and
R4 is -H or a group of the formula (V) 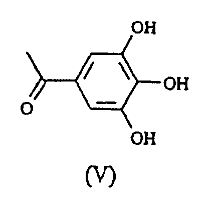 for the preparation of a pharmaceutical with at least 5% - 75% (w/w), preferably at least 10% - 60% (w/w), in particular at least 25% - 55% (w/w), and especially at least 35% - 50% (w/w) of the compound of the formula (I) for the treatment of viral skin diseases and/or tumor diseases caused by papilloma viruses.

2. The Use according to claim 1, **characterized in that,** independent of each other, the radical R₁ and/or the radical R₂ is/are substituted by a halogen, preferably fluorine and/or chlorine, an unbranched or branched C₁-C₆alkyl, alkylene or alkynyl radical, preferably a C₁-C₃alkyl, alkylene or alkynyl radical, in particular a methyl radical.

3. The use according to claim 1 or 2, **characterized in that** the radical A is derived from myristic acid.

4. The use according to claim 1 or 2, **characterized in that** the radical B is derived from ethanol, propanol, isopropanol, butanol, ethylene glycol, polyethylene glycol, propylene glycol, polypropylene glycol, glycerol, polyglycerol, arabitol, adonitol, xylitol, sorbitol, mannitol and/or dulcitol.

5. The use according to at least one of claims 1 - 4, **characterized in that** the compound (I) is isopropyl myristate.

6. The use according to at least one of claims 1 - 5, **characterized in that** the catechol is selected from epicatechol, epicatechol gallate, epigallocatechol, epigallocatechol gallate, gallocatechol and gallocatechol gallate, in particular (-)-epicatechol, (-)-epicatechol gallate, (-)-epigallocatechol, (-)-epigallocatechol gallate, (+)-gallocatechol and (-)-gallocatechol gallate.

7. The use according to at least one of claims 1 - 6, **characterized in that** catechols are present in the form of a mixture, preferably comprising 5,9%(w/w) of (-)-epicatechol, 12,6% (w/w) of (-)-epicatechol gallate, 17,6% (w/w) of (-)-epigallocatechol, 53,9% (w/w) of (-)-epigallocatechol gallate and 1,4% (w/w)of (-)-gallocatechol gallate.

8. The use according to at least one of claims 1 - 6, **characterized in that** catechols are present in the form of a mixture comprising 10.8% (w/w) of (-)-epicatechol, 6.5% (w/w) of (-)-epicatechol gallate, 9.2% (w/w) of (-)-epigallocatechol, 54.8% (w/w) of (-)-epigallocatechol gallate and 4.0% (w/w) of (-)-gallocatechol gallate.

9. The use according to at least one of claims 1 - 8, **characterized in that** said catechols are isolated from a tea extract.

10. The use according to at least one of claims 1 - 9, **characterized in that** the formulation comprises 1-30% (w/w), preferably 2-20% (w/w) and, in particular, 15-18% (w/w) of a catechol and at least 5-90% (w/w), preferably at least 10-70% (w/w), in particular at least 25-60% (w/w) and, especially, at least 35-50% (w/w) of the compound (I).

11. The use according to at least one of claims 1 - 10, **characterized in that** the pharmaceutical active compound is hydrophobic.

12. The use according to at least one of claims 1 - 11, comprising further additives and/or auxiliary substances.

13. The use according to claim 12, **characterized in that** the additives and/or auxiliary substances are hydrophobic and are preferably selected from petroleum jelly, wax, oleyl alcohol, propylene glycol monostearate and propylene glycol monopalmitostearate.

14. The use according to any of claims 1 - 13, **characterizes in that** the pharmaceutical comprises 35% (w/w) of isopropyl myristate, 15% (w/w) of at least one catechol, 24.5% (w/w) of petroleum jelly, 20% (w/w) of wax, 5% (w/w) of propylene glycol monostearate or propylene glycol monopalmitostearate and 0.5% (w/w) of oleyl alcohol.

15. The use of a pharmaceutical according to any of claims 1 - 14, **characterized in that** the viral skin diseases and/or tumor diseases are caused by human papilloma viruses, such as HPV 1, 2, 3, 4, 5, 6, 8, 9, 11, 12, 13, 14, 15, 16, 17, 18, 19-29, 31, 32, 34, 36-38, 46-50, 56, 58.

16. The use of a pharmaceutical according to claim 15, **characterized in that** the skin diseases are warts, genital warts, benign tumors of the skin and/or mucosa caused by papilloma viruses, for example verrucae plantares, verrucae vulgares, verrucae planae juveniles, epidermodysplasia verruciformis, Condylomata acuminata, Condylomata plana, bowenoid papulosis, papillomas on the larynx and oral mucosa or focal epithelial hyperplasia.

17. The use of a pharmaceutical according to at least one of claims 1 - 16, **characterized in that** the pharmaceutical is applied topically, in particular genitally or vaginally.

## Revendications

1. Utilisation d'un composé de formule générale (I) en tant que principe actif pharmaceutique,
A-B (I)
et d'au moins une catéchine de formule générale (IV), 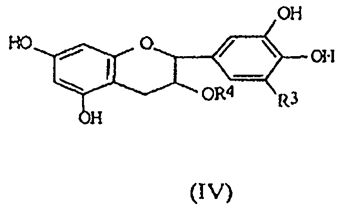 **caractérisée en ce que**, A représente un groupe de formule générale (II) 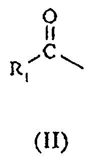 et B représente un groupe de formule générale (III)
-O-R₂ (III)
et
R₁ représente un groupe alkyle en C₁₃ et
R₂ représente indépendamment un groupe alkyle, alkylène ou alcynyle en C₁ à C₈ non ramifié ou ramifié, de préférence un groupe alkyle, alkylène ou alcynyle en C₁ à C₆, en particulier un groupe alkyle, alkylène ou alcynyle en C₂ à C₄, tout particulièrement un groupe alkyle en C₃, un groupe -[CH₂-(CH₂)ₘ-O]ₙ-H où n = 1 à 10, de préférence n = 1 à 5, m = 1 à 5, de préférence m = 1 à 3,
un groupe -CH₂-[CH-(OH)]ₚ-[CH₂-(R₃)]-, dans lequel R₃ représente indépendamment un atome d'hydrogène ou un groupe hydroxyle, p = 1 à 7, de préférence p = 1 à 4, un groupe pentose ou un groupe hexose, et
**en ce que**, dans la formule (IV),
R₃ représente -H ou -OH et
R₄ représente -H ou un groupe de formule (V) 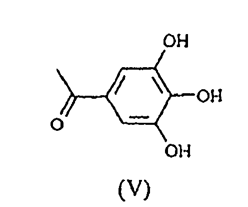 pour la préparation d'un médicament contenant au moins 5% à 75% (p/p), de préférence au moins 10% à 60% (p/p), en particulier au moins 25% à 55% (p/p) et tout particulièrement au moins 35% à 50% (p/p) du composé de formule générale (I) pour le traitement de maladies virales de la peau et/ou de maladies tumorales causées par des papillomavirus.

2. Utilisation selon la revendication 1, **caractérisée en ce que** le groupe R₁ et/ou le groupe R₂ sont substitués, indépendamment l'un de l'autre, par un atome d'halogène, de préférence de fluor et/ou de chlore, un groupe alkyle, alkylène ou alcynyle en C₁ à C₆ non ramifié ou ramifié, de préférence un groupe alkyle, alkylène ou alcynyle en C₁ à C₃, en particulier un groupe méthyle.

3. Utilisation selon la revendication 1 ou 2, **caractérisée en ce que** le groupe A est dérivé de l'acide myristique.

4. Utilisation selon la revendication 1 ou 2, **caractérisée en ce que** le groupe B est dérivé de l'éthanol, du propanol, de l'isopropanol, du butanol, de l'éthylèneglycol, du polyéthylèneglycol, du propylèneglycol, du polypropylèneglycol, de la glycérine, de la polyglycérine, de l'arabitol, de l'adonitol, du xylitol, du sorbitol, du mannitol et/ou du dulcitol.

5. Utilisation selon au moins l'une quelconque des revendications 1 à 4, **caractérisée en ce que** le composé (I) est le myristate d'isopropyle.

6. Utilisation selon au moins l'une quelconque des revendications 1 à 5, **caractérisée en ce que** la catéchine est choisie parmi l'épicatéchine, le gallate d'épicatéchine, l'épigallocatéchine, le gallate d'épigallocatéchine, la gallocatéchine et le gallate de gallocatéchine, en particulier l'(-)-épicatéchine, le gallate d'(-)-épicatéchine, l'(-)-épigallocatéchine, le gallate d'(-)-épigallocatéchine, la (+)-gallocatéchine et le gallate de (-)-gallocatéchine.

7. Utilisation selon au moins l'une quelconque des revendications 1 à 6, **caractérisée en ce que** les catéchines se présentent sous la forme d'un mélange, contenant de préférence 5,9% (p/p) d'(-)-épicatéchine, 12,6% (p/p) de gallate d'(-)-épicatéchine, 17,6% (p/p) d'(-)-épigallocatéchine, 53,9% (p/p) de gallate d'(-)-épigallocatéchine et 1,4% (p/p) de (-)-gallocatéchine.

8. Utilisation selon au moins l'une quelconque des revendications 1 à 6, **caractérisée en ce que** les catéchines se présentent sous la forme d'un mélange, contenant de préférence 10,8% (p/p) d'(-)-épicatéchine, 6,5% (p/p) de gallate d'(-)-épicatéchine, 9,2% (p/p) d'(-)-épigailocatéchine, 54,8% (p/p) de gallate d'(-)-épigallocatéchine et 4,0% (p/p) de gallate de (-)-gallocatéchine.

9. Utilisation selon au moins l'une quelconque des revendications 1 à 8, **caractérisée en ce que** les catéchines mentionnées sont obtenues à partir d'un extrait de thé.

10. Utilisation selon au moins l'une quelconque des revendications 1 à 9, **caractérisée en ce que** la formulation contient 1% à 30% (p/p), de préférence 2% à 20% (p/p) et en particulier 15% à 18% (p/p) d'une catéchine ainsi qu'au moins 5% à 90% (p/p), de préférence au moins 10% à 70% (p/p), en particulier au moins 25% à 60% (p/p) et tout particulièrement au moins 35% à 50% (p/p) du composé (I).

11. Utilisation selon au moins l'une quelconque des revendications 1 à 10, **caractérisée en ce que** le principe actif pharmaceutique est hydrophobe.

12. Utilisation selon au moins l'une quelconque des revendications 1 à 11, contenant d'autres additifs et/ou adjuvants.

13. Utilisation selon la revendication 12, **caractérisée en ce que** les additifs et/ou adjuvants sont hydrophobes et sont choisis de préférence parmi la vaseline, la cire, l'alcool oléylique, le monostéarate de propylèneglycol ou le monopalmitostéarate de propylèneglycol.

14. Utilisation selon l'une quelconque des revendications 1 à 13, **caractérisée en ce que** le médicament contient 35% (p/p) de myristate d'isopropyle, 15% (p/p) d'au moins une catéchine, 24,5% (p/p) de vaseline, 20% (p/p) de cire, 5% (p/p) de monostéarate de propylèneglycol ou de monopalmitostéarate de propylèneglycol et 0,5% (p/p) d'alcool oléylique.

15. Utilisation d'un médicament selon l'une quelconque des revendications 1 à 14, **caractérisée en ce que** les maladies virales de la peau et/ou les maladies tumorales sont causées par des papillomavirus humains tels que, par exemple, HPV 1, 2, 3, 4, 5, 6, 8, 9, 11, 12, 13, 14, 15, 16, 17, 18, 19-29, 31, 32, 34, 36-38, 46-50, 56, 58.

16. Utilisation d'un médicament selon la revendication 15, **caractérisée en ce que** les maladies de la peau sont des verrues, des verrues génitales, des tumeurs bénignes de la peau et/ou des muqueuses causées par des papillomavirus, par exemple des verrues plantaires, des verrues vulgaires, des verrues planes juvéniles, une épidermodysplasie verruciforme, des condylomes acuminés, des condylomes plans, des papuloses bowenoïdes, des papillomes au niveau du larynx et de la muqueuse buccale ou une hyperplasie épithéliale focale.

17. Utilisation d'un médicament selon au moins l'une quelconque des revendications 1 à 16, **caractérisée en ce que** le médicament est appliqué par voie topique, en particulier génitale ou vaginale.
